# EUROPÄISCHE PATENTSCHRIFT

(11) **EP 1 620 498 B1**
(45) Veröffentlichungstag und Bekanntmachung des Hinweises auf die Patenterteilung: **06.08.2008**
(21) Anmeldenummer: 04724078.3
(22) Anmeldetag: 29.03.2004
(51) Int. Cl.: C08J 11/10, C08C 19/08, C12R 1/00, C12N 1/20, B09B 3/00

(54) **VERFAHREN ZUR OBERFLÄCHENAKTIVIERUNG UND/ODER OBERFLÄCH ENDEVULKANISATION VON SCHWEFELVERNETZTEN GUMMIPARTIKELN**
PROCESS FOR SURFACE ACTIVATION AND/OR DEVULCANISATION OF SULPHUR-VULCANISED RUBBER PARTICLES
PROCEDE D'ACTIVATION ET/OU DE DEVULCANISATION DE LA SURFACE DE PARTICULES DE CAOUTCHOUC VULCANISEES AVEC DU SOUFRE

(30) Priorität: 01.04.2003 DE 10314893
(43) Veröffentlichungstag der Anmeldung: 01.02.2006
(73) Patentinhaber: Cristallo Holdings Inc., Edmonton / Alberta T5P4P4 (CA)
(72) Erfinder: NEUMANN, Willi, 04849 Bad Dueben (DE)
(74) Vertreter: Schröer, Gernot H.
(86) Internationale Anmeldenummer: PCT/IB2004/000932
(87) Internationale Veröffentlichungsnummer: WO 2004/087799

(56) Entgegenhaltungen:
- US-A- 5 518 619
- K. BREDBERG ET AL.: "Anaerobic desulfurization of ground rubber with the thermophilic archaeon Prococcus furiosus - a new method for rubber recycling" APPL. MICROBIOL. BIOTECHNOLOL., Bd. 55, 10. November 2000 (2000-11-10), Seiten 43-48, XP002294913 in der Anmeldung erwähnt

## Beschreibung

Die Erfindung betrifft ein Verfahren zur Oberflächenaktivierung und/oder Oberflächendevulkanisation von schwefelvernetzten Gummipartikeln.

Aus DE 4425049 C1, DE 19607281 A1, US 5,506,283 sind unterschiedliche Verfahren zur Aktivierung von zerkleinertem Altgummi bzw. Abfallgummi bekannt. Diese Verfahren basieren entweder auf physikalischen oder auf chemischen Wirkprinzipien bzw. auf einer Kombination beider Wirkprinzipien.

Ferner sind aus DE 4042009 C2, EP 0493732 B1, US 5,597,851 und DE 19728036 A1 Verfahren zur mikrobiellen bzw. enzymatischen Aktivierung von Gummimehlen und Gummigranulaten bekannt.

Aus US 5,518,619 ist ein Verfahren zur Entfernung von Schwefelverbindungen aus Wasser bekannt, bei dem die Schwefelverbindungen einer anaeroben Reduktion unterworfen werden.

In den Dokumenten DE 4042009 C2 und EP 0493732 B1 wird ein Verfahren beschrieben, das auf einer mikrobiellen Oxidation des polysulfidisch gebundenen Schwefels in Gummivulkanisaten beruht. Die Oxidation des polysulfidischen Schwefels an der Oberfläche der Gummipartikel erfolgt mit Hilfe von chemolithotrophen Mikroorganismen in einer Bakteriensuspension bei definierter Sauerstoffzufuhr. Die Bakterien gehören zur Gattung Thiobacillus. Die Oxidation des Schwefels erfolgt in der Regel bis zum Sulfat. Das Zielprodukt des Verfahrens ist ein replastiziertes schwefelarmes Gummimaterial mit guter Vulkanisationseignung.

Ein ähnliches Verfahren wird in US 5,597,851 beschrieben. Die Besonderheit dieses Verfahrens besteht einerseits darin, dass als schwefeloxidierender Mikroorganismus primär der thermophile fakultativ chemolithotrophe Sulfolobus acidocaldarius eingesetzt wird und zum anderen die Behandlung der Gummipartikel lediglich mit dem Enzymsystem dieses Mikroorganismus erfolgt. Die Gummipartikel selbst kontaktieren nicht unmittelbar mit den Mikroorganismen.

In DE 19728036 A1 wird ein Verfahren beschrieben, bei dem durch biotechnologische Behandlung von vulkanisierten Gummipartikeln durch definierte Reaktionszeiten/Oxidationsdauern bestimmte reaktive funktionelle Gruppen in Form von Hydroxylgruppen, Epoxygruppen und Carboxylgruppen an der Partikeloberfläche erzeugt werden. Dadurch wird es möglich, das aktivierte Gummimehl bzw. Gummigranulat mit unterschiedlichen Kunststoffen, Bitumen und anderen Polymeren zu vernetzen. Für die mikrobielle Oxidation werden gleichfalls Bakterien der Gattung Thiobacillus eingesetzt.

Die bisher bekannten Verfahren zur mikrobiellen Aktivierung von Gummimehlen bzw. Gummigranulaten durch Schwefeloxidation weisen folgende gravierenden Nachteile auf:
1. Diese Aktivierungsverfahren basieren auf Oxidationsprozessen. Neben der gewünschten Oxidation des polysulfidischen Schwefels erfolgt zwangsläufig eine unerwünschte simultane Oxidation der Polymerketten (Anlagerung von Radikalen). Die noch bindungsaktiven Stellen der Partikeloberfläche werden praktisch beseitigt. Der Grad der Schädigung hängt u.a. ab vom Gummityp (Anzahl der Doppelbindungen), der Reaktionstemperatur, der Reaktionsdauer und der Konzentration des gelösten Sauerstoffs in der Suspension.
2. Die Schädigung der Polymerketten bewirkt u.a. eine unerwünschte Freisetzung von bestimmten Gummiinhaltsstoffen (Weichmacher, Ruß, Zinkoxid, usw.).
3. Um Fremdinfektionen zu vermeiden, müssen die Prozesse bei sehr niedrigen pH-Werten (1 bis 3) betrieben werden, was zusätzliche Anforderungen an die Werkstoffe der Bioreaktoren und an die Abwasserbehandlung bedingt.

Diese Nachteile können durch anaerobe Verfahren vermieden werden. Aus Bredberg (K. Bredberg, J. Perssom, M. Christiansson, B. Stenberg, O. Holst: "Anaerobic desulfurization of ground rubber with the thermophilic archaeon Pyrococcus furiosus - a new method for rubber recycling" in der Zeitschrift Appl. Microbiol. Biotechnol. (2001) 55, Seiten 43-48) ist ein derartiges Verfahren unter Verwendung des schwefelreduzierenden, anaeroben, extrem thermophilen Archaeons Pyrococcus furiosus bekannt. Bei diesem Verfahren bestehen allerdings - insbesondere aufgrund der extrem thermophilen Eigenschaft des Archaeons - folgende Nachteile:
1. Die Behandlung des Gummimehles über einen längeren Zeitraum bei einem Temperaturniveau von 90 - 100° C führt zu einer Schädigung der Polymerketten der Elastomere und damit zu einer Verschlechterung der signifikanten werkstofftechnischen Parameter (Zugfestigkeit, Reißdehnung, Abrieb, usw.).
2. Durch die hohe Temperaturbelastung des Gummimehls werden verstärkt Gummiinhaltsstoffe freigesetzt (Weichmacher, Ruß, Zinkoxid, chemische Schutzstoffe, usw.), die toxisch auf die Mikroorganismen wirken und damit den Prozess der Entschwefelung hemmen bzw. zum Prozessabbruch führen.
3. Die Prozessführung bei einem derart hohen Temperaturniveau ist im Hinblick auf großtechnische Realisierungen unwirtschaftlich und ökologisch bedenklich (Freisetzung von toxischen Stoffen in das Prozessabwasser).

Der Erfindung liegt daher die Aufgabe zugrunde, ein Verfahren zur Oberflächenaktivierung und/oder Oberflächendevulkanisation von schwefelvernetzten Gummipartikeln anzugeben, das im Wesentlichen bei Temperaturen unterhalb von 90 °C abläuft und die oben angeführten Nachteile mikrobieller Oxidationsverfahren vermeidet.

Diese Aufgabe wird gemäß der Erfindung gelöst durch ein Verfahren mit den Merkmalen des Anspruchs 1. Vorteilhafte Weiterbildungen und Ausgestaltungen sind in den von Anspruch 1 abhängigen Ansprüchen angegeben.

Die Erfindung beruht auf der Überlegung, ein Verfahren zur Oberflächenaktivierung und/oder Oberflächendevulkanisation von schwefelvernetzten Gummipartikeln anzugeben, bei dem die Gummipartikel zum Aufbrechen der Schwefelbrücken und zur Reduktion des Schwefels biotechnologisch in einem Medium mit mesophilen anaeroben und/oder mesophilen fakultativ anaeroben und/oder mesophilen mikroaerophilen Bakterien und/oder einem oder mehreren Enzymsystemen dieser Bakterien behandelt werden. Unter fakultativ anaeroben Bakterien sind dabei Bakterien zu verstehen, die sowohl mit als auch ohne Sauerstoff auskommen.

Der wesentliche Unterschied zu dem in Bredberg beschriebenen Verfahren liegt in dem Einsatz mesophiler Mikroorganismen. Die optimalen Lebensbedingungen mesophiler Bakterien liegen bei 20 bis 45 °C. Somit funktioniert das erfindungsgemäße Verfahren bei Temperaturen deutlich unterhalb von 90 °C. Dadurch werden die oben beschriebenen Nachteile einer Behandlung mit extrem thermophilen Mikroorganismen beseitigen bzw. zumindest stark reduziert. Da es sich bei den im erfindungsgemäßen Verfahren eingesetzten Bakterien ferner um anaerobe und/oder fakultativ anaerobe und/oder mikroaerophile Bakterien handelt, funktioniert das Verfahren unter Ausschluss von Sauerstoff bzw. bei sehr geringen Sauerstoffkonzentrationen. Dadurch werden die oben beschriebenen Nachteile der mikrobiellen Oxidationsverfahren vermieden.

Das Wirkprinzip dieser Behandlung der Gummipartikel nach dem erfindungsgemäßen Verfahren besteht darin, dass Mikroorganismen die Schwefelbrücken des vulkanisierten Gummis an der Partikeloberfläche aufbrechen und den Schwefel teilweise oder vollständig reduzieren, ohne dabei die Polymerketten der Elastomere zu schädigen. Durch das erfindungsgemäße Verfahren werden aktivierte Gummipartikel erzeugen, die im Vergleich zu nicht aktivierten Gummipartikeln über ein hohes Vulkanisationsvermögen verfügen. Dadurch wird es möglich, unter Verwendung derartig aktivierter Gummipartikel, insbesondere in Form von Gummimehlen und Gummigranulaten, Produkte hoher Qualität zu erzeugen.

Die Behandlung der Gummipartikel bei dem Verfahren gemäß der Erfindung kann mikrobiell und/oder enzymatisch sein. Im Falle eines enzymatischen Verfahrens erfolgt die Behandlung insbesondere ausschließlich mit dem, vorzugsweise von den Bakterien isolierten, Enzymsystem der Bakterien.

Unter mikrobieller Behandlung wird dabei verstanden, dass die Bakterien selbst in Kontakt (Wechselwirkung) mit der Oberfläche der Gummipartikel treten. Bei einer enzymatischen Behandlung hingegen erfolgt die Behandlung, d.h. das Aufbrechen der Schwefelbrücken und/oder die Schwefelreduktion, nur mit dem bzw. den Enzymsystem(en) der Bakterien.

Im Einzelnen kann bei einer enzymatischen Behandlung folgendermaßen vorgegangen werden: Zunächst werden die Bakterien in einer Vorkultur ohne Kontakt mit den zu behandelnden schwefelvernetzten Gummipartikeln unter Verwendung eines anderen Schwefelsubstrates, beispielsweise elementarem Schwefel, vermehrt. Anschließend werden die Bakterien zerstört und das Enzymsystem wird durch allgemein bekannte Verfahren isoliert (geerntet). Die Behandlung der schwefelvernetzten Gummipartikel erfolgt dann ausschließlich mit dem isolierten Enzymsystem.

Es ist aber auch möglich, dass die Bakterien das Enzymsystem in situ produzieren, d.h. das für die enzymatische Behandlung erforderliche Enzymsystem wird nicht vorab isoliert.

Zweckmäßigerweise enthält das Medium zur Behandlung der Gummipartikel Wasser, Nährstoffe, Kohlenstoffquelle und Bakterien oder besteht daraus und ist damit eine Suspension. Ein vorteilhafte Weiterbildung sieht vor, dass die Konzentration des Gummipartikelmaterials im Medium im Wesentlichen unter 35 Massenprozent gehalten wird. Höhere Gummipartikelkonzentrationen bewirken Probleme bei der Durchmischung der Reaktionsmasse, bei der Stoffübertragung und beim Wachstum der Bakterien, beispielsweise aufgrund zu hoher Konzentrationen an toxischen chemischen Substanzen, insbesondere aus Alterungsschutzmitteln.

Ferner kann vorgesehen sein, dass das Medium zum Abbau von Temperatur- und/oder Konzentrationsgradienten durchmischt wird. Vorzugsweise wird die Durchmischung schonend vorgenommen, beispielsweise mittels eines Rührwerks.

Eine weitere zweckmäßige Variante des erfindungsgemäßen Verfahrens sieht vor, dass die Behandlung unter anaeroben oder mikroaerophilen Bedingungen erfolgt. Ferner kann vorgesehen sein, dass die Behandlung im Wesentlichen bei Temperaturen unterhalb von 90 °C, insbesondere unterhalb von 50 °C, vorzugsweise innerhalb eines für mesophile Bakterien optimalen Temperaturbereichs, der bei etwa 20 bis 45 °C liegt, erfolgt. Vorteilhafterweise erfolgt die Behandlung bei Temperaturen in einem Bereich von 33 bis 37°C.

Dadurch wird die Behandlung der Gummipartikel insgesamt unter Bedingungen vorgenommen, die optimal an die Lebensbedingungen der mesophilen anaeroben und/oder mesophilen fakultativ anaeroben und/oder mesophilen mikroaerophilen Bakterien angepasst sind. Ferner lassen sich dadurch weitestgehend die eingangs angesprochenen Nachteile der bekannten Verfahren verhindern.

Gemäß einer Weiterbildung des Verfahrens liegt der pH-Wert im Bereich von 5 bis 9, insbesondere von 6 bis 8. Ferner kann die Verweilzeit der Gummipartikel im Bereich von 4 bis 8 Tagen, insbesondere von 5 bis 7 Tagen, vorzugsweise bei etwa 6 Tagen, liegen.

Optimale Entschwefelungserfolge wurden insbesondere erzielt unter Einhaltung folgender Prozessparameter und Bedingungen:

| | |
|---|---|
| Prozesstemperatur: | 33 bis 37°C |
| pH-Wert: | 6 bis 8 |
| Durchmischungsregime: | schonende Durchmischung mit Hilfe eines Rührwerks |
| Mittlere Verweilzeit: | 6 Tage |
| Korngröße der Gummipartikel: | 0,2 bis 0,4 mm |

Eine zweckmäßige Weiterbildung des Verfahrens gemäß der Erfindung sieht vor, dass es sich bei den in dem Medium und/oder zur Produktion des Enzymsystems eingesetzten Bakterien um zur Schwefelatmung, d.h. Schwefelreduktion, befähigte Bakterien handelt.

In Untersuchungen wurde festgestellt, dass nach einer entsprechenden Adaptationsphase unterschiedliche anaerobe oder fakultativ anaerobe oder mikroaerophile mesophile Bakterien in der Lage sind, die Schwefelbrücken im vulkanisierten Gummi zu brechen und den Schwefel zu reduzieren. Positive Resultate wurden u.a. erzielt mit den Bakterien Desulfuromonas thiophila, Desulfuromonas palmitatis, Sulfurospirillum deleyianum und Desulfuromonas acetoxidans. Vorteilhafterweise werden daher Bakterien eingesetzt, die im Wesentlichen einem oder mehreren dieser Bakterienstämme angehören. Ferner kann es sich bei allen oder einigen der Bakterien um Mischpopulationen handeln.

Sehr gute Entschwefelungsraten ergaben sich durch den Einsatz einer anaeroben, mesophilen Mischpopulation, die neben den schwefelreduzierenden Bakterien über signifikante Anteile methanogener Bakterien verfügt. Diese Population wurde aus einem Flusssediment (Saale) isoliert und zeichnet sich durch eine besondere Stabilität aus.

Eine Ausgestaltung der Erfindung sieht vor, dass es sich bei den zu behandelten Gummipartikeln im Wesentlichen um ein Gummipulver und/oder ein Gummimehl und/oder ein Gummigranulat handelt. Unter Gummipulver und Gummigranulat ist dabei ein Material mit einem Partikeldurchmesser von weniger als 1 mm zu verstehen, unter Gummigranulat ein Material mit einem Partikeldurchmesser zwischen ca. 1 mm und 5 mm. Zweckmäßig und vorteilhaft ist, wenn die Partikelgröße der zu behandelnden Gummipartikel im Bereich von 0,1 bis 0,6 mm, insbesondere von 0,2 bis 0,4 mm, liegt, d.h. wenn es sich um Gummipulver bzw. Gummimehl handelt.

Zweckmäßigerweise ist gemäß einer Weiterbildung vorgesehen, dass die zu behandelten Gummipartikeln im Wesentlichen Gummipartikel aus schwefelvernetzten Gummitypen oder aus Verbundwerkstoffen auf Grundlage von schwefelvernetzen Gummitypen sind. Das erfindungsgemäße Verfahren ist grundsätzlich für die Oberflächenaktivierung und/oder Oberflächendevulkanisation aller schwefelvernetzten Gummitypen, z.B. SBR (Styrol/Butadien-Elastomar), NR (Naturgummi), NBR (Acrylnitril/Butadien-Elastomer, Nitrilgummi) und EPDM (Ethylen/Propylen/Dien-Elastomer), geeignet.

Gemäß einer vorteilhaften Weiterbildung sind die Gummipartikel aus Altgummi (z.B. Altreifen, technische Gummierzeugnisse wie Dichtungen, Profile, Gummiformteile, Förderbänder) und/oder Abfallgummi (Produktionsabfälle der gummierzeugenden und gummiverarbeitenden Industrie) entstanden. Auf diese Weise dient das erfindungsgemäße Verfahren zur Aufbereitung von Alt- und/oder Abfallgummi.

Eine weitere Ausführungsform sieht vor, dass die zu behandelnden Gummipartikel in einem Zerkleinerungsverfahren hergestellt sind, insbesondere einem Schälverfahren und/oder einer Warmvermahlung und/oder einer Kaltvermahlung und/oder einer kryogenen Mahlung und/oder einer Nassvermahlung. Besonders vorteilhaft ist dabei, wenn bei dem Zerkleinerungsverfahren zur Herstellung der Gummipartikel die Temperatur der Gummipartikel so niedrig bleibt, insbesondere im Wesentlichen kleiner als 90 °C, dass eine thermooxidative Degradation der Gummipartikel weitestgehend vermieden wird.

Eine besonders vorteilhafte Weiterbildung des erfindungsgemäßen Verfahrens sieht vor, dass die Oberflächenaktivierung und/oder Oberflächendevulkanisation im Wesentlichen auf die Gummipartikeloberfläche und/oder oberflächennahe Schichten beschränkt ist, um die werkstofflichen Eigenschaften der Hauptmasse des Gummipartikelmaterials nicht zu verändern. Die oberflächennahen Schicht sollte dabei höchstens 300 nm dick sein. Dies bedeutet, dass die Wirkung der mesophilen Entschwefelung bewusst auf die Partikeloberfläche und/oder oberflächennahen Schichten beschränkt wird.

Zweckmäßigerweise erfolgt die Behandlung der Gummipartikel in einem Bioreaktor. Ein Bioreaktor ist ein Apparat zur reproduzierbaren und kontrollierten Durchführung von Stoffumwandlungen mit Mikroorganismen. Ferner kann vorgesehen sein, dass die Zugabe der zu behandelnden Gummipartikel in den Bioreaktor und/oder die Entnahme der behandelten Gummipartikel aus dem Bioreaktor kontinuierlich oder quasikontinuierlich oder diskontinuierlich erfolgt. Alternativ oder additiv kann ferner vorgesehen sein, dass der Bioreaktor so betrieben wird, dass bei der Entnahme der behandelten Gummipartikel aus dem Bioreaktor keine oder nur geringe Mengen des Bakterien und/oder Enzyme enthaltenden Mediums zur Behandlung der Gummipartikel mit ausgetragen werden und/oder mit Luftsauerstoff in Berührung kommen. Dies kann erreicht werden durch Sedimentation und anschließende Ausschleusung des Gummipartikelmaterials unter anaeroben Bedingungen.

Zweckmäßigerweise werden bei dem Verfahren die in den Gummipartikeln enthaltenen Schwefelbrücken durch die Behandlung wenigstens teilweise aufgebrochen und der Schwefel in ein oder mehrere gasförmige Reaktionsprodukte übergeführt. Eines der gasförmigen Reaktionsprodukte kann Schwefelwasserstoff sein. Eine besonders vorteilhafte Weiterentwicklung sieht vor, dass der bei der Behandlung der Gummipartikel gebildete Schwefelwasserstoff kontinuierlich oder quasikontinuierlich aus der Gasphase ausgeschleust wird. Dadurch lässt sich eine Hemmungen und/oder Vergiftungen der Bakterien vermeiden.

Eine vorteilhafte Weiterbildung des Verfahrens gemäß der Erfindung sieht vor. dass die behandelten Gummipartikel nach der Behandlung insbesondere zur Verringerung einer Salzlast mit Wasser gewaschen und anschließend schonend getrocknet werden, insbesondere im Wesentlichen bei Temperaturen unter 90° C.

Eine weitere Ausgestaltung sieht vor, dass durch die Behandlung oberflächenaktivierte Gummipartikel, insbesondere Gummimehle, erzeugt werden, die zur Herstellung von Gummiprodukten verwendet werden. Dabei können diese neuen Gummiprodukte entweder im Wesentlichen nur aus behandelten, oberflächenaktivierten Gummipartikeln oder aus den oberflächenaktivierten Gummipartikeln und zugemischtem Frischgummi, insbesondere durch chemische Vernetzung, hergestellt werden.

Ferner kann vorgesehen sein, dass durch die Behandlung oberflächenaktivierte Gummipartikel, insbesondere Gummimehle, erzeugt werden, die zur Herstellung von Elastomerlegierungen, insbesondere durch Phasenkopplung mit Kunststoffen, vorzugsweise Polypropylen (PP) und/oder Polyurethan (PU), verwendet werden.

Neben der Verbesserung der werkstofflichen Eigenschaften der auf diese Weise hergestellten Gummiprodukte bewirkt die Verwendung derart oberflächenaktivierter Gummipartikel auch eine Reduktion der spezifischen Produktkosten.

Beispielsweise führt die Zumischung eines nach dem Verfahren gemäß der Erfindung aktivierten Altgummimehls zu Frischkautschuk im Vergleich zur Zumischung von unbehandelten Mehlen zu einer signifikanten Verbesserung der werkstofftechnischen Parameter des sich ergebenden Produkts, insbesondere des Spannungs-Dehnungsverhaltens, des Weiterreißwiderstandes und der Rückprallelastizität. Ferner lässt sich feststellen, dass durch Compoundieren von derart aktivierten Altreifenmehlen und EPDM-Mehlen mit Thermoplasten - insbesondere mit Polypropylenen - Werkstoffe entstehen, deren mechanisch-physikalischen Eigenschaften denen von thermoplastischen Elastomeren nahe kommen. Insbesondere ist eine Verbesserung der Elastizität im Vergleich zum Einsatz von vergleichbaren unbehandelten Altgummimehlen festzustellen. Dies deutet darauf hin, dass es zu einer intensiven Interdiffusion der Ketten der Polymerphase und der Elastomerphase und wahrscheinlich auch zu einer chemischen Vernetzung beider Phasen kommt (intensive Phasenkopplung).

Im folgenden wird die Erfindung anhand eines Ausführungsbeispiels weiter erläutert.

Ein kryogen gemahlenes EPDM-Gummimehl, mit einer Korngröße kleiner 0,4 mm, wird unter anaeroben Bedingungen mikrobiell oberflächendevulkanisiert. Bei einer Aktivierungsdauer von 8 Tagen wird ein Entschwefelungsgrad des Gummis von ca. 4 % erreicht. Das mikrobiell aktivierte Gummimehl und nicht aktiviertes Gummimehl der gleichen Ausgangsprobe werden jeweils mit EPDM-Frischgummi im Verhältnis 1 : 1 gemischt und vulkanisiert.

Die Zugfestigkeit und die Reißdehnung der jeweiligen Endprodukte sowie - zum Vergleich - von EPDM-Frischgummi sind in folgender Tabelle dargestellt:

| Endprodukt vulkanisiert aus: | Zugfestigkeit in MPa | Reißdehnung in % |
|---|---|---|
| EPDM-Frischgummi | 28 | 595 |
| (ohne Gummimehlzumischung) | | |
| 50 % EPDM-Frischgummi | 25 | 555 |
| + 50 % aktiviertes Gummimehl | | |
| 50 % EPDM-Frischgummi | 17,5 | 385 |
| + 50 % nicht aktiviertes Gummimehl | | |

Der Vergleich der angegebenen Werte für die signifikanten Werkstoffparameter Zugfestigkeit und Reißdehnung zeigt eindeutig, dass eine erfindungsgemäße Behandlung von Gummipartikeln, d.h. im betrachteten Beispiel eine mikrobiell unter anaeroben Bedingungen durchgeführte Aktivierung von Gummimehl, zu einer erheblichen Verbesserung der Werkstoffeigenschaften im Vergleich zu nicht behandelten Gummipartikeln führt.

Ingesamt weisen somit die gemäß dem Verfahren nach der Erfindung behandelten, aktivierten Gummipartikel im Vergleich zu unbehandelten Gummipartikeln ein verbessertes Vulkanisationsverhalten auf und gestattet dadurch die Herstellung hochwertiger Produkte.

## Patentansprüche

1. Verfahren zur Oberflächenaktivierung und/oder Oberflächendevulkanisation von schwefelvernetzten Gummipartikeln, bei dem die Gummipartikel zum Aufbrechen der Schwefelbrücken und zur Reduktion des Schwefels biotechnologisch in einem Medium mit mesophilen anaeroben und/oder mesophilen fakultativ anaeroben und/oder mesophilen mikroaerophilen Bakterien und/oder einem oder mehreren Enzymsystemen dieser Bakterien behandelt werden.

2. Verfahren nach Anspruch 1, bei dem die Behandlung der Gummipartikel mikrobiell und/oder enzymatisch erfolgt, insbesondere ausschließlich mit dem, vorzugsweise von den Bakterien isolierten, Enzymsystem erfolgt.

3. Verfahren nach einem der vorhergehenden Ansprüche, bei dem das Medium zur Behandlung der Gummipartikel Wasser und Nährstoffe und Kohlenstoffquelle und Bakterien enthält oder daraus besteht und/oder die Konzentration des Gummipartikelmaterials im Medium unter 35 Massenprozent gehalten wird.

4. Verfahren nach einem der vorhergehenden Ansprüche, bei dem zum Abbau von Temperatur- und/oder Konzentrationsgradienten das Medium durchmischt wird, insbesondere eine schonende Durchmischung, vorzugsweise mittels eines Rührwerks, erfolgt.

5. Verfahren nach einem der vorhergehenden Ansprüche, bei dem die Behandlung unter anaeroben oder mikroaerophilen Bedingungen erfolgt.

6. Verfahren nach einem der vorhergehenden Ansprüche, bei dem die Behandlung bei Temperaturen unterhalb von 90 °C, insbesondere unterhalb von 50 °C, vorzugsweise innerhalb eines für mesophile Bakterien optimalen Temperaturbereichs, insbesondere von 33 bis 37°C, erfolgt.

7. Verfahren nach einem der vorhergehenden Ansprüche, bei dem die Behandlung bei einem pH-Wert im Bereich von 5 bis 9, insbesondere von 6 bis 8, erfolgt.

8. Verfahren nach einem der vorhergehenden Ansprüche, bei dem Verweilzeit der Gummipartikel im Medium im Bereich von 4 bis 8 Tagen, insbesondere von 5 bis 7 Tagen, vorzugsweise bei etwa 6 Tagen, liegt.

9. Verfahren nach einem der vorhergehenden Ansprüche, bei dem die Bakterien zur Schwefelatmung, d.h. Schwefelreduktion, befähigte Bakterien sind oder umfassen, insbesondere einem oder mehreren der folgenden Bakterienstämme angehören: Desulfuromonas thiophila, Desulfuromonas palmitatis, Sulfurospirillum deleyianum, Desulfuromonas acetoxidans.

10. Verfahren nach einem der vorhergehenden Ansprüche, bei dem die Bakterien Mischpopulationen sind oder umfassen.

11. Verfahren nach einem der vorhergehenden Ansprüche, bei dem die zu behandelten Gummipartikel ein Gummipulver und/oder ein Gummimehl und/oder ein Gummigranulat sind oder umfassen, wobei die Partikelgröße vorzugsweise im Bereich von 0,1 bis 0,6 mm, insbesondere von 0,2 bis 0,4 mm, liegt..

12. Verfahren nach einem der vorhergehenden Ansprüche, bei dem die zu behandelten Gummipartikeln Gummipartikel aus schwefelvernetzten Gummitypen oder aus Verbundwerkstoffen auf Grundlage von schwefelvernetzen Gummitypen sind oder umfassen.

13. Verfahren nach einem der vorhergehenden Ansprüche, bei dem die zu behandelten Gummipartikeln Gummipartikel aus Altgummi und/oder Abfallgummi sind oder umfassen und das Verfahren damit zur Aufbereitung von Alt- und/oder Abfallgummi dient.

14. Verfahren nach einem der vorhergehenden Ansprüche, bei dem die zu behandelnden Gummipartikel in einem Zerkleinerungsverfahren hergestellt sind, insbesondere einem Schälverfahren und/oder einer Warmvermahlung und/oder einer Kaltvermahlung und/oder einer kryogenen Mahlung und/oder einer Nassvermahlung, wobei vorzugsweise die Temperatur der Gummipartikel so niedrig bleibt, insbesondere kleiner als 90 °C, dass eine thermooxidative Degradation der Gummipartikel weitestgehend vermieden wird.

15. Verfahren nach einem der vorhergehenden Ansprüche, bei dem die Oberflächenaktivierung und/oder Oberflächendevulkanisation im Wesentlichen auf die Gummipartikeloberfläche und/oder oberflächennahe Schichten, insbesondere mit einer Dicken von höchstens 300 nm, beschränkt ist, um die werkstofflichen Eigenschaften der Hauptmasse des Gummipartikelmaterials nicht zu verändern.

16. Verfahren nach einem der vorhergehenden Ansprüche, bei dem die Behandlung der Gummipartikel in einem Bioreaktor erfolgt.

17. Verfahren nach Anspruch 16, bei dem die Zugabe der zu behandelnden Gummipartikel in den Bioreaktor und/oder die Entnahme der behandelten Gummipartikel aus dem Bioreaktor kontinuierlich oder quasikontinuierlich oder diskontinuierlich erfolgt und/oder der Bioreaktor so betrieben wird, dass bei der Entnahme der behandelten Gummipartikel aus dem Bioreaktor keine oder nur geringe Mengen des Bakterien und/oder Enzyme enthaltenden Mediums zur Behandlung der Gummipartikel mit ausgetragen werden und/oder mit Luftsauerstoff in Berührung kommen, insbesondere durch Sedimentation des Gummipartikelmaterials und dessen anschließende Ausschleusung unter anaeroben Bedingungen.

18. Verfahren nach einem der vorhergehenden Ansprüche, bei dem die in den Gummipartikeln enthaltenen Schwefelbrücken durch die Behandlung wenigstens teilweise aufgebrochen und der Schwefel in ein oder mehrere gasförmige Reaktionsprodukte übergeführt wird, wobei insbesondere eines der gasförmigen Reaktionsprodukte Schwefelwasserstoff ist, der vorzugsweise zur Vermeidung von Hemmungen und/oder Vergiftungen der Bakterien kontinuierlich oder quasikontinuierlich aus der Gasphase ausgeschleust wird.

19. Verfahren nach einem der vorhergehenden Ansprüche, bei dem die behandelten Gummipartikel nach der Behandlung insbesondere zur Verringerung einer Salzlast mit Wasser gewaschen und anschließend schonend getrocknet werden, insbesondere bei Temperaturen unter 90°C.

20. Verfahren nach einem der vorhergehenden Ansprüche, bei dem durch die Behandlung oberflächenaktivierte Gummipartikel, insbesondere Gummimehle, erzeugt werden, die zur Herstellung von Gummiprodukten verwendet werden, die insbesondere nur aus den behandelten, oberflächenaktivierten Gummipartikeln, oder aus den oberflächenaktivierten Gummipartikeln und zugemischtem Frischgummi, hergestellt werden.

21. Verfahren nach einem der vorhergehenden Ansprüche, bei dem durch die Behandlung oberflächenaktivierte Gummipartikel, insbesondere Gummimehle, erzeugt werden, die zur Herstellung von Elastomerlegierungen, insbesondere durch Phasenkopplung mit Kunststoffen, vorzugsweise Polypropylen (PP) und/oder Polyurethan (PU), verwendet werden.

## Claims

1. Process for surface activation and/or surface vulcanisation of sulphur-vulcanised rubber particles, wherein, in order to break the sulphur bridges and to reduce the sulphur, the rubber particles are treated in a biotechnological manner in a medium with mesophilic anaerobic and/or mesophilic optionally anaerobic and/or mesophilic microaerophilic bacteria and/or with one or more enzyme systems of such bacteria.

2. Process according to Claim 1, wherein the treatment of the rubber particles is microbial and/or enzymatic, in particular involving exclusively the enzyme system preferably isolated from the bacteria.

3. Process according to one of the preceding claims, wherein the medium for treating the rubber particles contains water and nutrients and carbon source and bacteria or consists thereof and/or the concentration of the rubber particle material is held in the medium below 35 mass per cent.

4. Process according to one of the preceding claims, wherein, in order to reduce temperature and/or concentration gradients, the medium is mixed thoroughly, in particular a mild mixing, preferably by means of an agitator, occurs.

5. Process according to one of the preceding claims, wherein the treatment occurs under anaerobic or microaerophilic conditions.

6. Process according to one of the preceding claims, wherein the treatment occurs at temperatures below 90 °C, in particular below 50 °C, preferably within an optimum temperature range for mesophilic bacteria, in particular from 33 to 37 °C.

7. Process according to one of the preceding claims, wherein the treatment occurs with a pH value in the range from 5 to 9, in particular from 6 to 8.

8. Process according to one of the preceding claims, wherein the residence time of the rubber particles in the medium is in the range of 4 to 8 days, in particular of 5 to 7 days, preferably at around 6 days.

9. Process according to one of the preceding claims, wherein the bacteria for sulphur aspiration, i.e. sulphur reduction, are or comprise empowered bacteria, in particular belong to one or more of the following bacterial strains: Desulfuromonas thiophilia, Desulfuromonas palmitatis, Sulfurospirillum deleyianum, Desulfuromonas acetoxidans.

10. Process according to one of the preceding claims, wherein the bacteria are or comprise mixed populations.

11. Process according to one of the preceding claims, wherein the rubber particles being treated are or comprise a rubber powder and/or a rubber flour and/or rubber granulate, wherein the particle size is preferably in the range of 0.1 to 0.6 mm, in particular from 0.2 to 0.4 mm.

12. Process according to one of the preceding claims, wherein the rubber particles being treated are or comprise rubber particles made of sulphur-vulcanised rubber types or composite materials based on sulphur-vulcanised rubber types.

13. Process according to one of the preceding claims, wherein the rubber particles being treated are or comprise rubber particles made of scrap rubber and/or waste rubber and the process thus serves the recovery of scrap and/or waste rubber.

14. Process according to one of the preceding claims, wherein the rubber particles being treated are manufactured in a comminution process, in particular a peeling process and/or a hot grinding and/or a cold grinding and/or a cryogenic grinding and/or a wet grinding, wherein preferably the temperature of the rubber particles remains so low, in particular lower than 90 °C, that a thermooxidative degradation of the rubber particles is avoided as far as possible.

15. Process according to one of the preceding claims, wherein the surface activation and/or surface vulcanisation is essentially limited to the rubber particle surface and/or layers close to the surface, in particular with a thickness of at most 300 nm, so as not to alter the material properties of the main mass of the rubber particle material.

16. Process according to one of the preceding claims, wherein the treatment of the rubber particles occurs in a bioreactor.

17. Process according to Claim 16, wherein the addition to the bioreactor of the rubber particles being treated and/or the removal of the treated rubber particles from the bioreactor occurs continuously or quasi-continuously or discontinuously and/or the bioreactor is operated such that, with the removal of the treated rubber particles from the bioreactor, no or only slight quantities of the bacteria and/or enzyme-containing medium for treating the rubber particles are simultaneously discharged and/or come into contact with atmospheric oxygen, in particular by sedimentation of the rubber particle material and its subsequent discharge under anaerobic conditions.

18. Process according to one of the preceding claims, wherein the sulphur bridges contained in the rubber particles are at least partially broken by the treatment and the sulphur is converted into one or more gaseous reaction products, wherein in particular one of the gaseous reaction products is hydrogen sulphide, which is preferably discharged continuously or quasi-continuously from the gas phase to prevent inhibition and/or toxication of the bacteria.

19. Process according to one of the preceding claims, wherein the treated rubber particles after treatment, in particular to reduce a saline load, are washed with water and then gently dried, in particular at temperatures below 90 °C.

20. Process according to one of the preceding claims, wherein, through the treatment, surface-activated rubber particles, in particular rubber flours, are generated which are used for the manufacture of rubber products, which in particular are manufactured from the treated, surface-activated rubber particles, or from the surface-activated rubber particles and added fresh rubber.

21. Process according to one of the preceding claims, wherein, through the treatment, surface-activated rubber particles, in particular rubber flours, are generated which are used for the manufacture of elastomer alloys, in particular by phase coupling with plastics, preferably polypropylene (PP) and/or polyurethane (PU).

## Revendications

1. Procédé d'activation superficielle et/ou de dévulcanisation de la surface de particules de caoutchouc ponté par le soufre, dans lequel, pour dissocier les ponts soufre et pour réduire le soufre, les particules de caoutchouc sont traitées par voie biotechnologique dans un milieu contenant des bactéries mésophiles anaérobies et/ou mésophiles anaérobies facultatives et/ou mésophiles micro-aérophiles et/ou contenant un ou plusieurs systèmes enzymatiques issus de ces bactéries.

2. Procédé selon la revendication 1, dans lequel le traitement des particules de caoutchouc se fait par voie microbienne et/ou enzymatique, en particulier en utilisant exclusivement le système enzymatique isolé des bactéries.

3. Procédé selon l'une des revendications précédentes, dans lequel le milieu de traitement des particules de caoutchouc contient ou se compose d'eau et de substances nutritives et d'une source de carbone et de bactéries et/ou la concentration du matériau des particules de caoutchouc dans le milieu est maintenue à moins de 35% en masse.

4. Procédé selon l'une des revendications précédentes, dans lequel, pour supprimer les gradients de température et/ou de concentration, le milieu est mélangé, en particulier mélangé intimement avec ménagements, de préférence au moyen d'un agitateur.

5. Procédé selon l'une des revendications précédentes, dans lequel le traitement se fait en conditions anaérobies ou micro-aérophiles.

6. Procédé selon l'une des revendications précédentes, dans lequel le traitement se fait à des températures inférieures à 90°C, en particulier inférieures à 50°C, de préférence dans une plage de température optimale pour les bactéries mésophiles, en particulier dans la plage de 33 à 37°C.

7. Procédé selon l'une des revendications précédentes, dans lequel le traitement se fait à un pH dans la plage de 5 à 9, en particulier de 6 à 8.

8. Procédé selon l'une des revendications précédentes, dans lequel le temps de séjour des particules de caoutchouc dans le milieu va de 4 à 8 jours, en particulier de 5 à 7 jours, et il est de préférence d'environ 6 jours.

9. Procédé selon l'une des revendications précédentes, dans lequel les bactéries sont ou comprennent des bactéries capables de respirer le soufre, c'est-à-dire de le réduire, en particulier celles appartenant à une ou plusieurs des souches de bactéries suivantes: *Desulfuromonas thiophila*, *Desulfuromonas palmitatis, Sulfurospirillum deleyianum, Desulfuromonas acetoxidans*.

10. Procédé selon l'une des revendications précédentes, dans lequel les bactéries sont ou comprennent des populations mixtes.

11. Procédé selon l'une des revendications précédentes, dans lequel les particules de caoutchouc à traiter sont ou comprennent une poudre de caoutchouc et/ou une farine de caoutchouc et/ou un granulé de caoutchouc dont la granulométrie se situe de préférence dans la plage de 0,1 à 0,6 mm, en particulier de 0,2 à 0,4 mm.

12. Procédé selon l'une des revendications précédentes, dans lequel les particules de caoutchouc à traiter sont ou comprennent des particules de caoutchouc constituées de types de caoutchouc ponté par le soufre ou de matériaux composites à base de types de caoutchouc ponté par le soufre.

13. Procédé selon l'une des revendications précédentes, dans lequel les particules de caoutchouc à traiter sont ou comprennent des particules de caoutchouc de récupération et/ou de déchets de caoutchouc et le procédé sert donc à retraiter le caoutchouc de récupération et/ou les déchets de caoutchouc.

14. Procédé selon l'une des revendications précédentes, dans lequel les particules de caoutchouc à traiter sont obtenues par un procédé de broyage, en particulier par un procédé de déchiquetage et/ou un broyage à chaud et/ou un broyage à froid et/ou un broyage cryogénique et/ou un broyage humide, de préférence en maintenant la température à une valeur suffisamment basse, en particulier inférieure à 90°C, pour éviter le plus possible une dégradation thermo-oxydative des particules de caoutchouc.

15. Procédé selon l'une des revendications précédentes, dans lequel l'activation superficielle et/ou la dévulcanisation de surface se limite essentiellement à la surface des particules de caoutchouc et/ou aux couches proches de la surface, en particulier ayant une épaisseur maximale de 300 nm, de façon à ne pas modifier les propriétés matérielles de la masse principale du matériau des particules de caoutchouc.

16. Procédé selon l'une des revendications précédentes, dans lequel le traitement des particules de caoutchouc se fait dans un bioréacteur.

17. Procédé selon la revendication 16, dans lequel l'introduction des particules de caoutchouc à traiter dans le bioréacteur et/ou la récupération des particules de caoutchouc traitées à la sortie du bioréacteur se fait de manière continue ou quasi continue ou discontinue et/ou la conduite du bioréacteur se fait de telle manière que, lors de la récupération des particules de caoutchouc traitées à la sortie du bioréacteur, des quantités nulles ou minimes du milieu contenant les bactéries et/ou les enzymes servant à traiter les particules de caoutchouc sont extraites en même temps et/ou mises en contact avec l'oxygène de l'air, en particulier par une sédimentation du matériau des particules de caoutchouc suivie de son évacuation en conditions anaérobies.

18. Procédé selon l'une des revendications précédentes, dans lequel les ponts soufre contenus dans les particules de caoutchouc sont dissociés au moins en partie par le traitement et le soufre est converti en un ou plusieurs produits réactionnels gazeux, l'un desdits produits réactionnels gazeux étant notamment l'hydrogène sulfuré, qui est de préférence évacué de manière continue ou quasi continue de la phase gazeuse afin d'éviter des inhibitions et/ou des empoisonnements des bactéries.

19. Procédé selon l'une des revendications précédentes, dans lequel, après traitement, les particules de caoutchouc traitées sont lavées à l'eau, en particulier pour réduire la charge en sel, et ensuite séchées avec ménagements, en particulier à des températures inférieures à 90°C.

20. Procédé selon l'une des revendications précédentes, dans lequel sont produites des particules de caoutchouc, en particulier des farines de caoutchouc, activées en surface par le traitement qui sont utilisées pour fabriquer des produits en caoutchouc, obtenus en particulier uniquement à partir des particules de caoutchouc traitées ou à partir des particules de caoutchouc activées en surface additionnées de caoutchouc frais.

21. Procédé selon l'une des revendications précédentes, dans lequel sont produites des particules de caoutchouc, en particulier des farines de caoutchouc, activées en surface par le traitement qui sont utilisées pour produire des alliages élastomères, en particulier par couplage de phases avec des matières plastiques, de préférence du polypropylène (PP) et/ou du polyuréthane (PU).
